# EUROPEAN PATENT SPECIFICATION

(11) **EP 4 063 867 B1**
(45) Date of publication and mention of the grant of the patent: **06.08.2025**
(21) Application number: 21165012.2
(22) Date of filing: 25.03.2021
(51) Int. Cl.: G01N 33/94, G01N 27/48

(54) **METHOD FOR DETERMINING THE PRESENCE OF AN ANALYTE**
VERFAHREN ZUR BESTIMMUNG DES VORHANDENSEINS EINES ANALYTEN
PROCÉDÉ POUR DÉTERMINER LA PRÉSENCE D'UN ANALYTE

(43) Date of publication of application: 28.09.2022
(73) Proprietor: Universiteit Antwerpen, 2000 Antwerpen (BE)
(72) Inventor: DE WAEL, Karolien, 9170 Sint-Pauwels (BE); DAEMS, Devin, 2850 Boom (BE); PARRILLA PONS, Marc, 2018 Antwerpen (BE); DE JONG, Mats, 2250 Olen (BE)
(74) Representative: Winger

(56) References cited:
- EP-A1- 3 698 131
- EP-A1- 3 786 643
- MATS DE JONG ET AL: "Electrochemical fingerprint of street samples for fast on-site screening of cocaine in seized drug powders", CHEMICAL SCIENCE, vol. 7, no. 3, 1 January 2016 (2016-01-01), United Kingdom, pages 2364 - 2370, XP055428519, ISSN: 2041-6520, DOI: 10.1039/C5SC04309C

## Description

### Technical field of the invention

The present invention relates to a method for determining the presence of an analyte in a sample.

### Background of the invention

A compound, such as an analyte, may have a well-defined voltammetric signal, for example at which the analyte is oxidized or reduced. The presence of the voltammetric signal of an analyte in a voltammetric response of a sample may therefore confirm the presence of the analyte in the sample. Determining whether an analyte is present in a sample may, however, be complicated when an interferant is present in the sample. For example, a voltammetric signal of the interferant may partially overlap with the voltammetric signal of the analyte. Thereby, it may be unclear whether a voltammetric signal corresponds to that of the analyte or to that of the interferant. Furthermore, the presence of the interferant may influence the voltage at which the voltammetric signal of the analyte occurs, for example, because of the overlap. As a result, the voltammetric signal of the analyte in a first sample that is a mixture comprising the analyte and the interferent may be shifted with respect to the voltammetric signal of the analyte in a second sample comprising the analyte but not comprising the interferant.
Also, when the sample is analyzed outside of a controlled environment, e.g. a laboratory, some parameters of the sample are difficult to control. For example, the sample temperature may be difficult to keep within boundaries that are compatible with the analytical method used. It may be desirable to determine the presence of an analyte, e.g., a narcotic, in a sample suspected of comprising the analyte. Preferably, this determination is performed immediately when the sample is found, that is, at the location where the sample was found. For example, by immediately determining the presence of the analyte, it may be possible to take immediate action. At the location where the sample is found, the temperature of the environment and the sample temperature may range from very cold at night and in the winter, to very hot in a container on a summer day. The voltammetric signal of the analyte and of interferants may strongly depend on the sample temperature. Thereby, a voltammetric signal of the voltammetric response of the sample corresponding to that of the analyte may, erroneously, not be recognized as corresponding to that of the analyte, which may result in a false-negative determination of the presence of the analyte. In contrast, a voltammetric signal corresponding to that of an interferant may, erroneously, be attributed to the analyte, which may result in a false-positive determination of the presence of the analyte.

EP3786643A1 discloses the use of a source of formaldehyde for methylating a primary or secondary amine as part of an electrochemical measurement for detection of primary or secondary amines such as drugs, antibiotics and amino acids, and an electrode coated with formaldehyde or its adduct.

De Jong, M., Sleegers, N., Kim, J., Van Durme, F., Samyn, N., Wang, J., & De Wael, K. (2016). Electrochemical fingerprint of street samples for fast on-site screening of cocaine in seized drug powders. Chemical science, 7(3), 2364-2370 discloses a wearable fingertip sensor for on-the-spot identification of cocaine and its cutting agents in street samples. They present the distinct voltammetric response of cocaine, cutting agents, binary mixtures of cocaine and street samples in solution and powder street samples.

There is a need in the art for good methods that may yield accurate determination of the presence of the analyte in a sample also outside of a controlled environment.

### Summary of the invention

It is an object of the present invention to provide a good method for determining the presence of an analyte in a sample at a sample temperature.

The above objective is accomplished by a method and apparatus according to the present invention. The invention is as defined in the claims.

It is an advantage of embodiments of the present invention that the presence of the analyte in the sample may be determined in environments at various temperatures. It is an advantage of embodiments of the present invention that the sample does not need to be transferred to a laboratory to accurately determine the presence of the analyte in the sample. It is an advantage of embodiments of the present invention that the presence of the analyte may be determined over a wide temperature range. It is an advantage of embodiments of the present invention that the method may be accurate. It is an advantage of embodiments of the present invention that the chance of a false-positive or false-negative determination of the analyte in the sample may be low. It is an advantage of embodiments of the present invention that the method may be fast.

In a first aspect, the present invention relates to a method for determining the presence of an analyte in a sample at a sample temperature, the method comprising: selecting a reference voltage range amongst at least a first reference voltage range and a second reference voltage range, wherein the selected reference voltage range is associated with a temperature range in which the sample temperature falls, wherein the first reference voltage range is associated with a first temperature range and the second reference voltage range is associated with a second temperature range, each reference voltage range comprising a plurality of different voltage values at which a voltammetric signal of the analyte can be detected at a temperature falling in the temperature range associated with the reference voltage range, measuring a voltammetric response of the sample at least across the selected reference voltage range, and determining whether the analyte is present in the sample by analyzing whether the voltammetric signal of the analyte can be detected in the measured voltammetric response.

In a second aspect, the present invention relates to a data processing apparatus adapted for carrying out the steps of the method according to embodiments of the first aspect.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change, and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

FIG. 1 is a plot of a plurality of voltammetric responses of a sample comprising cocaine, measured at different temperatures.
FIG. 2 is a plot of the peak voltage of the voltammetric signal of a reference cocaine sample measured at different temperatures.
FIG. 3 is a plot of a voltage range, for each of ten compounds, in which a peak voltage of the voltammetric signal of the compound may fall.
FIG. 4 is a plot of the peak voltage of the voltammetric signal of a reference diltiazem sample measured at different temperatures.
FIG. 5 is a plot of the peak voltage of the voltammetric signal of a reference hydroxyzine sample measured at different temperatures.
FIG. 6 is a plot of the peak voltage of the voltammetric signal of a reference ketamine sample measured at different temperatures.
FIG. 7 is a plot of the peak voltage of the voltammetric signal of a reference 3,4-methylenedioxymethamphetamine sample measured at different temperatures.
FIG. 8 is a plot of the peak voltage of the voltammetric signal of a reference amphetamine sample measured at different temperatures.
FIG. 9 is a plot of the peak voltage of the voltammetric signal of a reference heroin sample measured at different temperatures.
FIG. 10 is a plot of the peak voltage of the voltammetric signal of a reference methamphetamine sample measured at different temperatures.
FIG. 11 is a plot of a peak voltage of the voltammetric response at a sample temperature for each of 173 samples comprising cocaine, for determining the presence of cocaine in the sample, in accordance with an embodiment of the present invention.
FIG. 12 is a plot of a peak voltage of the voltammetric response at a sample temperature for each of 173 samples comprising cocaine, for determining the presence of cocaine in the sample, using a method not in accordance with an embodiment of the present invention.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps, or components as referred to, but does not preclude the presence or addition of one or more other features, integers, steps or components, or groups thereof. The term "comprising" therefore covers the situation where only the stated features are present and the situation where these features and one or more other features are present. The word "comprising" according to the invention therefore also includes as one embodiment that no further components are present. Thus, the scope of the expression "a device comprising means A and B" should not be interpreted as being limited to devices consisting only of components A and B. It means that with respect to the present invention, the only relevant components of the device are A and B.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures, or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures, and techniques have not been shown in detail in order not to obscure an understanding of this description.

In a first aspect, the present invention relates to a method for determining the presence of an analyte in a sample at a sample temperature, the method comprising: selecting a reference voltage range amongst at least a first reference voltage range and a second reference voltage range, wherein the selected reference voltage range is associated with a temperature range in which the sample temperature falls, wherein the first reference voltage range is associated with a first temperature range and the second reference voltage range is associated with a second temperature range, each reference voltage range comprising a plurality of different voltage values at which a voltammetric signal of the analyte can be detected at a temperature falling in the temperature range associated with the reference voltage range, measuring a voltammetric response of the sample at least across the selected reference voltage range, and determining whether the analyte is present in the sample by analyzing whether the voltammetric signal of the analyte can be detected in the measured voltammetric response.

Typically, before selecting a reference voltage range, at least a first reference voltage range and a second reference voltage range are provided.

Preferably, before selecting a reference voltage range, at least a first reference voltage range and a second reference voltage range are provided in an electronic memory, and selecting a reference voltage range comprises retrieving the reference voltage range in the electronic memory.

In embodiments, the sample temperature is the temperature of the sample at the moment the voltammetric response is measured. In embodiments, the sample temperature is measured using a temperature sensor contacting the sample. This is advantageous because the sample temperature may thereby be determined very accurately. In embodiments, the sample temperature is estimated by measuring the temperature of an environment of the sample, e.g., of air within ten meters, preferably within 1 meter, more preferably within 10 centimeters of the sample. In embodiments, the temperature sensor may be comprised in a data processing apparatus according to the second aspect. In embodiments, the temperature of the sample is assumed to be identical to the temperature of the environment of the sample. It is an advantage of these embodiments that determining the sample temperature may be very straightforward. However, the sample temperature is not required to be derived from measurements using temperature sensors, and instead, a sample temperature may, for instance, be estimated by a person performing the method, or may be derived from meteorological data.

In embodiments, the analyte may comprise any compound of interest. In embodiments, the analyte comprises an explosive compound or a narcotic, preferably a narcotic. In embodiments, the explosive compound is selected from the list consisting of 2,4,6-Trinitrotoluene (TNT), 1,3,5-Trinitrobenzene (TNB), triacetone triperoxide (TATP), pentaerythritol tetranitrate (PETN), 1,3,5-trinitro-1,3,5- triazacyclohexane (RDX), 3,4,8,9,12,13-Hexaoxa-1,6-diazabicyclo[4.4.4]tetradecane (HMTD), preferably TNT, RDX, and TNB.

In embodiments, the narcotic is one of the following compounds: cocaine, heroin, amphetamine, methamphetamine, 3,4-methylenedioxymethamphetamine, and ketamine. In preferred embodiments, the narcotic is cocaine.

In embodiments, the sample is a solution, preferably comprising water as a solvent. It is an advantage of embodiments of the present invention that water may be a good solvent for many analytes, such as narcotics. Furthermore, water may advantageously be buffered, so that the pH of the sample may be accurately controlled. In embodiments, a powder suspected of comprising the analyte is dissolved in the solvent, e.g., water, to form the sample. The sample may comprise the analyte and/or interferants, but the invention is not limited thereto.

As disclosed in EP3698131A1, a voltammetric signal of an analyte, e.g., a narcotic, in a sample may be better resolved from a voltammetric signal of an interferant when the sample is at a first pH than when the sample is at a second pH. For example, some interferants may have a voltammetric signal overlapping with, or suppressing the voltammetric signal of the narcotic at the second pH, but not at the first pH. In embodiments, the narcotic is cocaine, amphetamine or ketamine, and the pH of the sample is from 10 to 13. It is an advantage of embodiments of the present invention that a voltammetric signal of these narcotics may be well-resolved from a voltammetric signal of interferants in this pH range. The invention is however not limited thereto, and these narcotics may instead be measured at a different pH. In embodiments, the narcotic is amphetamine and the pH is from 9 to 13. In these embodiments, the preferred pH may depend on the environment of amphetamine during measurement. In presence of 1,2-naphthoquinone-4-sulphonic acid sodium (NQS), in a hydrogen carbonate buffer, pH 10 is preferred. In presence of formaldehyde, pH 12 is preferred. In embodiments, the sample may comprise a buffer. This is advantageous because the buffer may help control the pH of the sample. In embodiments, the sample comprises a hydrogen carbonate buffer or phosphate-buffered saline (PBS). In alternative embodiments, the narcotic is 3,4-methylenedioxymethamphetamine (MDMA), and the pH of the sample is from 4 to 6. It is an advantage of embodiments of the present invention that a voltammetric signal of MDMA may be well-resolved from a voltammetric signal of interferants in this pH range. In embodiments, the sample comprises a sodium acetate buffer.

In embodiments, measuring the voltammetric response of the sample comprises contacting the sample with a working electrode and a counter electrode. In embodiments, measuring the voltammetric response comprises applying a voltage to the working electrode and measuring a current through the working electrode. In embodiments, measuring the voltammetric response comprises measuring the current through the working electrode as dependent on applied voltage over a voltage range, wherein the voltage range at least comprises the selected reference voltage range.

In embodiments, any type of working and reference electrode may be used to measure the voltammetric response. In embodiments, the working and/or the reference electrode used to measure the voltammetric response is a screen-printed carbon electrode. It is an advantage of embodiments of the present invention that screen-printed carbon electrodes may require only a small sample volume, e.g. a few microliters, to perform the method. In embodiments, 20 microliters or less may be enough. It is an advantage of embodiments of the present invention that the sensitivity of screen-printed carbon electrodes may be comparatively high. In embodiments, a reference electrode is used that is preferably a silver electrode.

In embodiments, the voltammetric response may be measured using any suitable voltammetric technique, such as cyclic voltammetry, differential pulse voltammetry, or square wave voltammetry. In preferred embodiments, the voltammetric response is measured using square wave voltammetry. This is advantageous because square wave voltammetry may be faster and more sensitive than alternative voltammetric techniques. In embodiments, the square wave voltammetry may be performed with a step potential, i.e., a voltage resolution, of from 1 to 10 mV (e.g., 5 mV). In embodiments, the square wave voltammetry may be performed with a frequency of from 1 to 50 Hz, preferably 2 to 30 Hz, more preferably from 5 to 15 Hz (e.g., 10 Hz). In embodiments, the square wave voltammetry may be performed with an amplitude of 5 to 100 mV, preferably from 10 to 50 mV, more preferably from 15 to 40 mV, yet more preferably 20 to 30 mV (e.g., 25 mV).

In embodiments, before measuring the voltammetric response, before or after contacting the sample with a working electrode used to measure the voltammetric response, a pretreatment is applied at a pretreatment potential to the working electrode for at least 5 seconds, preferably at least 60 seconds, yet more preferably at least 200 seconds (e.g., 360 s). In general, and specifically for cocaine, the pretreatment potential may measure between -0.4 V and -2 V versus Ag/AgCl, preferably between -0.5 V and -1.2 V. For heroine, the pretreatment may be from 0.5 to 2.5 V versus Ag/AgCl, preferably between 1.0 and 2.0 V, more preferably between 1.3 and 1.7 V (e.g. 1.5 V). It is an advantage of embodiments of the present invention that, as is disclosed in EP3698131A1, such a pretreatment may result in better resolved voltammetric signals of a sample, e.g., of a narcotic. Thereby, the voltammetric signal of the analyte, e.g., the narcotic, may be better resolved from the voltammetric signal of an interferant.

In embodiments, each of the plurality of different voltage values at which a voltammetric signal of the analyte can be detected corresponds to a voltage value at which the voltammetric signal of the analyte is nonzero. In embodiments, a current through the working electrode that is due to the analyte, on the application of a voltage corresponding to the voltage value, is nonzero. In an embodiment, the voltage value may be any voltage at which a voltammetric signal can be detected. In preferred embodiments, each of the plurality of different voltage values corresponds to a peak voltage of the voltammetric signal of the analyte, and analyzing comprises determining whether a peak voltage of the measured voltammetric response falls within the selected reference voltage range. This is advantageous because the voltammetric signal is largest, i.e., has a maximum, at the peak of the voltammetric signal, so that the voltammetric signal is relatively easier to measure at the peak voltage. A peak voltage may be straightforwardly distinguished in a voltammetric response and in a voltammetric signal. It is an advantage of embodiments of the present invention that a peak voltage may be located at a predictable voltage, that is, the peak voltage may be reproducible between different measurements. In an embodiment, the peak voltage of the measured voltammetric response corresponds to the voltage at which a peak, i.e., a current peak, occurs in the measured voltammetric response. In embodiments, the peak voltage of the measured voltammetric response may correspond to a maximum, such as a local maximum or a global maximum, in the measured voltammetric response. In embodiments, the peak voltage of the voltammetric signal corresponds to the voltage at which a peak, i.e., a current peak, occurs in the voltammetric signal. In embodiments, the peak voltage of the voltammetric signal may correspond to a maximum, such as a local maximum or a global maximum, in the voltammetric signal. In embodiments, a current peak corresponds to a local or global maximum in the current.

The plurality of different voltage values forms a continuum of voltage values. Each voltage value is a voltage value at which the analyte can be detected at a temperature falling in the temperature range associated with the reference voltage range. Each temperature falling in the temperature range is typically associated with a sub-range of voltage values within the reference voltage range. At a specific temperature, the analyte can produce a voltammetric signal falling at different voltages depending on factors such as the analyte concentration or the presence of interferents in the sample.

For some analytes, for example ketamine, heroin, and 3,4-methylenedioxymethamphetamine, the voltammetric signal of the analyte may comprise a first and a second voltage range wherein the voltammetric signal i.e. current is nonzero. For example, the analyte may be oxidized a first time in the first voltage range and be oxidized a second time in the second voltage range. In embodiments, the method may comprise that a further reference voltage range is selected amongst at least a first further reference voltage range and a second further reference voltage range, wherein the selected further reference voltage range is associated with a further temperature range in which the sample temperature falls, wherein the first further reference voltage range is associated with a first further temperature range and the second further reference voltage range is associated with a second further temperature range, each further reference voltage range comprising a plurality of different further voltage values at which a voltammetric signal of the analyte can be detected at a temperature falling in the further temperature range associated with the further reference voltage range. In these embodiments, measuring may comprise that a voltammetric response of the sample is measured at least across the selected further reference voltage range. In embodiments, each of the plurality of different further voltage values corresponds to a further peak voltage of the voltammetric signal of the analyte, and analyzing comprises determining whether a further peak voltage of the measured voltammetric response falls within the selected further reference voltage range. Advantageously, at least for some analytes for which the voltammetric signal comprises a peak voltage and a further peak voltage, the presence of the analyte in a sample may be even more accurately determined, e.g. confirmed by the further peak voltage.

In embodiments, determining whether the analyte is present in the sample may be performed by a computer system. For example, the computer system may use a peak recognition software. Depending on the specifics of the situation, a number of criteria are available to the skilled person to decide whether or not the voltammetric signal of the analyte is resolved from a voltammetric signal of an interferant. One parameter in selecting an appropriate criterion may, for example, be a relative intensity between two voltammetric signals. If two peaks are of comparable intensity (e.g. the intensity of the peak maxima do not differ by more than 20%) a suitable criterion can be to consider two peaks as resolved if the separation between the two peak maxima is at least as large as their full width at half maximum (FWHM). A different criterion that may be used (e.g. when the peak maxima differ by more than 20%) is to consider two peaks as resolved if the separation between the two peak maxima is at least 50 mV. Further methods are for example discussed by Wang et al. (Wang, Joseph, and Bassam Freiha. "Evaluation and improvement of the resolution of voltammetric measurements." Talanta 33.5 (1986): 397-400).

The voltammetric response of the sample is at least measured over the selected voltage range. However, the voltammetric response may be measured over a much broader range, as long as at least the selected voltage range is measured. In embodiments, the presence of the analyte is determined by analyzing whether the voltammetric signal of the analyte is present in the selected voltage range. In embodiments, if the voltammetric signal of the analyte is present in the selected voltage range, it is concluded that the analyte is present, or likely present, in the sample. In embodiments, if the voltammetric signal of the analyte is not present in the selected voltage range, it is concluded that the analyte is not present, or likely not present, in the sample.

The voltammetric signal of an analyte in a sample may depend on the sample temperature. Usually, a relationship is assumed to exist between the voltage of the voltammetric signal of an analyte in a sample, e.g. the peak voltage, and the sample temperature. For example, the Nernst equation comprises a linear relationship between a temperature of a sample comprising a compound e.g. an analyte, and an oxidation or reduction voltage of the compound. Unexpectedly, the inventors have found that the relationship between the sample temperature and the voltage of the voltammetric signal e.g. the peak voltage may instead be nonlinear. The inventors have found that in some cases, the voltage of the voltammetric signal of an analyte in a sample, as dependent on the temperature of the sample, may not be accurately fitted with a linear curve, nor with a lower-order (e.g. second or third order) polynomial equation. Without being bound by theory, it is believed that interactions at the surface of the working electrode between an analyte and the surface may influence the reduction and/or oxidation voltage of the analyte. Thereby, extrapolation or interpolation of temperature-dependent data of a voltage at which the voltammetric signal occurs, e.g. a peak voltage, may be highly unreliable. Therefore, a method using an equation that relates a voltage at which the voltammetric signal occurs, e.g., a peak voltage of an analyte, to a temperature of a sample may have low accuracy, and may result in a false-positive or false-negative determination of the presence of the analyte in a sample. Also, the voltammetric signal may depend on the concentration of the analyte, and on the presence of interferants.

The inventors have found that the presence of an analyte may be accurately determined in a sample when, instead of equations, a plurality of reference voltage ranges, each associated with a different temperature range, is used to predict the voltage at which the voltammetric signal of the analyte occurs, i.e., to determine the presence of the analyte. By making use of different reference voltage ranges for different temperature ranges, the influence of a shift of the voltammetric signal as a result of a temperature difference may be limited.

The shift of a voltammetric signal in function of the temperature of an analyte may generally be in a same direction, which may be towards higher or lower voltage, as the shift of a voltammetric signal in function of the temperature of an interferant. In embodiments wherein the analyte is a narcotic, preferably cocaine, each reference voltage range associated with a temperature range is adapted so that the voltammetric signal of each of the interferants levamisole, phenacetin, lidocaine, caffeine, paracetamol, diltiazem, hydroxyzine, procaine, and benzocaine, in a sample at a sample temperature falling in the temperature range, does not fall in the reference voltage range. Advantageously, although a voltammetric signal of each of the interferants may be at a different voltage for each temperature range, the voltammetric signal of the interferant may not fall in a reference voltage range associated with the temperature range.

In embodiments, each of the plurality of different voltage values is a voltage, such as a peak voltage, at which a signal of the analyte can be observed in the associated temperature range. In embodiments, the plurality of different voltage values comprises discrete voltage values, such as those derived from experiments. In embodiments, the plurality of different voltage values forms a continuum of voltage values, e.g. so that the plurality of different voltage values comprise an infinite number of different voltage values. Typically, the continuum of voltage values comprises discrete voltage values derived from experiments.

In embodiments, at least some of the different voltage values within the plurality are determined by measuring a reference voltammetric response of a reference sample comprising the analyte.

In embodiments, each of the plurality of different voltage values is a voltage at which a voltammetric signal attributed to the analyte occurs in the reference voltammetric response at at least one temperature within the associated temperature range, in at least one chemical environment (presence or absence of one or more interferants), in at least one concentration.

In preferred embodiments, the reference voltage ranges, each associated with a different temperature range, are determined from the voltage values derived from reference voltammetric responses of the reference sample measured as dependent on temperature. In these embodiments, for each measurement, a same reference sample is preferably used. Advantageously, by using the same reference sample for each measurement, only the effect of sample temperature on the voltammetric signal is determined, isolated from other possible effects.

In preferred embodiments, preferably when a single reference sample is used for determined the voltage values, each of the plurality of different voltage values falling within a reference voltage range falls in a core reference voltage range associated with the reference voltage range. In embodiments, the core reference voltage range falls within the reference voltage range it is associated with. In embodiments, the core reference voltage range has a difference between an upper limit and a lower limit of from 10 mV to 50 mV, such as from 20 to 40 mV.

In embodiments, when a difference between a first voltage value and a second voltage value is larger than the difference between the upper limit and the lower limit, the first voltage value falls in a first core reference voltage range falling in a first reference voltage range associated with a first temperature range, and the second voltage value falls in a second core reference voltage range falling in a second reference voltage range associated with a second temperature range. This is advantageous as, within each temperature range associated with a reference voltage range, the shift of the voltammetric signal of the analyte due to a change in sample temperature is limited. Thereby, a width of the reference voltage range may be limited, as a shift of the voltammetric signal due to the temperature may be partly neglected in choosing the width of the reference voltage range. For example, even though a shift of a voltammetric signal of an analyte may be large as dependent on temperature, this may not result in a wide reference voltage range since, at a different temperature, a different reference voltage range may be selected.

An accessory aspect of the present invention relates to a process for constructing a set of reference voltage ranges comprising at least a first reference voltage range and a second reference voltage range. This process can, in some embodiments, form a first step of the method for determining the presence of an analyte according to the first aspect. However, typically, the process is performed separately from the method and leads to the construction of at least a first reference voltage range and a second reference voltage range that can then be used indefinitely in the method without having to ever perform the process again.

The process comprises the steps of:
a) measuring reference voltammetric responses of a reference sample at a first concentration and at a plurality of temperatures, the reference sample comprising the analyte, said plurality of temperatures including a first temperature lower or equal to 10°C and a second temperature higher or equal to 30°C,
b) forming from a first and a second peak voltage in the reference voltammetric responses respectively determined at said first and second temperatures an initial range of voltage values having said peak voltages as boundaries,
c) forming from the first and the second temperature an initial temperature range associated with the initial range of voltage values,
d) constructing a set of core reference voltage ranges by splitting the initial range of voltage values in a plurality of non-overlapping ranges, wherein at least all but one voltage ranges forming said plurality are 10 to 50 mV wide, and none of said plurality is wider than 50 mV,
e) constructing a set of core reference temperature ranges by splitting the initial temperature range at temperatures at which the peak voltages corresponding to the boundaries of the core reference voltage ranges occur,
f) constructing a set of partially overlapping reference voltage ranges by broadening each core reference voltage range obtained in d so as to include a voltage value corresponding to a peak voltage for the analyte in presence of at least one interferant, and a voltage value corresponding to a peak voltage for the analyte at a second concentration, different from the first concentration.

In embodiments, the reference sample comprises a solvent such as water, possibly comprising a buffer. The reference sample comprises the analyte. In embodiments, the reference sample used in step a of the accessory aspect comprises an interferant concentration that is lower than 10 mol-%, such as lower than 1 mol-%, preferably lower than 0.1 mol-%, even more preferably lower than 0.01 mol-%, of the concentration of the analyte. This is advantageous because this permits to obtain a core set of voltage values that are not influenced by the presence of interferants. In embodiments, step f of the accessory aspect comprises measuring voltammetric responses of a reference sample comprising the interferant at an interferant concentration that is from 10 mol-% to 200 mol-% that of the concentration of the analyte. This is advantageous as it allows an effect of the interferant on the voltammetric signal of the analyte to be determined.

In embodiments, the concentration of the analyte in the reference sample is from 0.1 mM to 3 mM, such as from 0.2 mM to 1.5 mM. However, the invention is not limited thereto, and instead, the concentration of the analyte may be larger or smaller. In embodiments, a difference between the pH of the reference sample and the pH of the sample is smaller than 3, such as smaller than 2, preferably smaller than 1.

In embodiments, each of the plurality of voltage values is determined by voltammetric measurements on the same reference sample. However, the invention is not limited to voltage values derived from a single reference sample. In embodiments, the voltage values included in step f may be derived from voltammetric response of a plurality of reference samples. For example, the plurality of reference samples may comprise at least one sample with a different concentration of the analyte than the reference sample and/or at least one sample comprising an interferant.

The width of the reference voltage range, that is, the difference between the upper and lower limit of the reference voltage range, is preferably chosen such that both false-positive and false-negative determinations of the presence of an analyte in a sample are prevented. The voltammetric signal of an analyte in a sample may be shifted with respect to the voltammetric signal of the analyte in the reference sample. For example, the shift may be due to a difference in concentration or the presence of interferants. In embodiments, an upper limit of the reference voltage range is higher, such as from 10 mV to 100 mV, preferably from 20 to 60 mV higher, than an upper limit of a core reference voltage range associated with the reference voltage range. In embodiments, a lower limit of the reference voltage range is lower, such as from 10 mV to 100 mV, preferably from 10 to 40 mV lower, than a lower limit of a core reference voltage range associated with the reference voltage range. In embodiments, a difference between an upper limit and a lower limit of each of the reference voltage ranges is from 30 mV to 200 mV, preferably from 60 mV to 120 mV, even more preferably from 80 mV to 100 mV. In embodiments, the differences are equal for each of the reference voltage ranges, but the invention is not limited thereto. The inventors have found that, in these embodiments, the reference voltage range may be sufficiently large to prevent a false-negative detection. The inventors have found that, in these embodiments, the reference voltage range may be sufficiently small to prevent a false-positive detection. Advantageously, both false-positive and false-negative detection of an analyte in a sample may be prevented.

In embodiments wherein the analyte is cocaine, selecting a reference voltage range is performed amongst a first, a second, and a third reference voltage range, wherein the first reference voltage range is associated with a first temperature range having an upper limit of from 11.5°C to 13.5°C, and possibly having a lower limit of from 0°C to 5°C, wherein the second reference voltage range is associated with a second temperature range having a lower limit higher but contiguous to the upper limit of the first temperature range and an upper limit of from 28.0°C to 30.0°C, and wherein the third reference voltage range is associated with a third temperature range having a lower limit higher but contiguous to the upper limit of the second temperature range, and possibly having a higher limit of from 35°C to 45°C. In these embodiments, the first reference voltage range has a lower limit of from 0.834 to 0.862 V and an upper limit of from 0.924 to 0.952 V, the second reference voltage range has a lower limit of from 0.794 V to 0.832 V and an upper limit of from 0.894 V to 0.922 V, and the third reference voltage range has a lower limit of from 0.774 V to 0.802 V and an upper limit of from 0.864 V to 0.892 V. In these embodiments, preferably, a working electrode and a counter electrode used for the voltammetric measurements both comprise a screen-printed carbon electrode, and a reference electrode used for the voltammetric measurements comprises a silver electrode. In these embodiments, preferably, the sample comprises water as a solvent, and the pH of the sample is from 10 to 13. The inventors have found, from a plurality of experiments on cocaine and possible interferants, that these reference voltage ranges and associated temperature ranges provide an accurate and reliable determination of the presence of cocaine in a sample, also when interferants are present in the sample, over a wide temperature range, and over a broad analyte concentration range.

In embodiments, the reference voltage ranges cover a temperature range of from 10 °C to 30 °C, preferably of from 1 °C to 40. It is an advantage of embodiments of the method of the first aspect that it may be accurate over a wide range of sample temperatures. It is a further advantage of embodiments of the method of the first aspect that it may be accurate even outside of a controlled, environment such as a laboratory.

In embodiments, preferably in embodiments wherein the analyte comprises a narcotic, each reference voltage range is an oxidation voltage range. Typically, voltammetric signals of narcotics are at an oxidation voltage. However, the invention is not limited thereto. In embodiments, preferably in embodiments wherein the analyte comprises an explosive compound, each reference voltage range is a reduction voltage range. Typically, voltammetric signals of explosive compounds are at a reduction voltage.

In embodiments, the method further comprises determining whether an interferant is present in the sample, comprising selecting an interferant reference voltage range amongst at least a first interferant reference voltage range and a second interferant reference voltage range, wherein the selected interferant reference voltage range is associated with an interferant temperature range in which the sample temperature falls, wherein the first interferant reference voltage range is associated with a first interferant temperature range and the second interferant reference voltage range is associated with a second interferant temperature range, each interferant reference voltage range comprising a plurality of different interferant voltage values at which a voltammetric signal of the interferant can be detected at a temperature falling in the interferant temperature range associated with the interferant reference voltage range, measuring the voltammetric response of the sample across the selected interferant reference voltage range, and determining whether the interferant is present in the sample by analyzing whether the voltammetric signal of the interferant can be detected in the measured voltammetric response. Typical interferants for narcotics comprise levamisole, phenacetin, lidocaine, caffeine, paracetamol, diltiazem, hydroxyzine, procaine, and benzocaine. It is an advantage of embodiments of the method of the first aspect that, by also determining whether interferants are present in the sample, purity of the sample may be estimated. It is an advantage of embodiments of the method of the first aspect that determining the presence of interferants may yield information on the source of the sample. Any features of the interferant reference voltage range may be independently as correspondingly described for the analyte reference voltage range.

Any features of any embodiment of the first aspect may be independently as correspondingly described for any embodiment of any of the other aspects of the present invention.

In a second aspect, the present invention relates to a data processing apparatus adapted for carrying out the steps of the method according to embodiments of the first aspect.

In embodiments, the data processing apparatus may comprise:
- an inlet for a sample,
- a unit for acquiring the temperature of the sample,
- a memory for storing at least a first reference voltage range, a second reference voltage range, and temperature ranges associated therewith,
- a selection unit for selecting a reference voltage range, amongst those stored in the memory, associated with a temperature range in which the sample temperature falls,
- a voltametric unit for measuring a voltametric response of the sample,
- an analyzing unit for determining whether the analyte is present in the sample by analyzing whether the voltammetric signal of the analyte can be detected in the measured voltammetric response,

In embodiments, the unit for acquiring the temperature of the sample may be a temperature sensor, a receiver for receiving the temperature from a temperature sensor, or an input unit allowing a user to enter the temperature (e.g., manually or vocally).

Any features of any embodiment of the second aspect may be independently as correspondingly described for any embodiment of any of the other aspects of the present invention.

In a third aspect, the present invention relates to a computer program comprising instructions which, when the program is executed by a computer, cause the computer to carry out the steps of the method according to embodiments of the first aspect of the present invention.

Any features of any embodiment of the third aspect may be independently as correspondingly described for any embodiment of any of the other aspects of the present invention.

In a fourth aspect, the present invention relates to a computer-readable medium comprising instructions which, when executed by a computer, cause the computer to carry out the steps of the method according to embodiments of the first aspect.

Any features of any embodiment of the fourth aspect may be independently as correspondingly described for any embodiment of any of the other aspects of the present invention.

In a fifth aspect, the present invention relates to a use of a sample temperature of a sample to select a reference voltage range for determining the presence of a narcotic in the sample.

Any features of any embodiment of the fifth aspect may be independently as correspondingly described for any embodiment of any of the other aspects of the present invention.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

### Example 1: Temperature-dependence of the voltammetric signal of cocaine

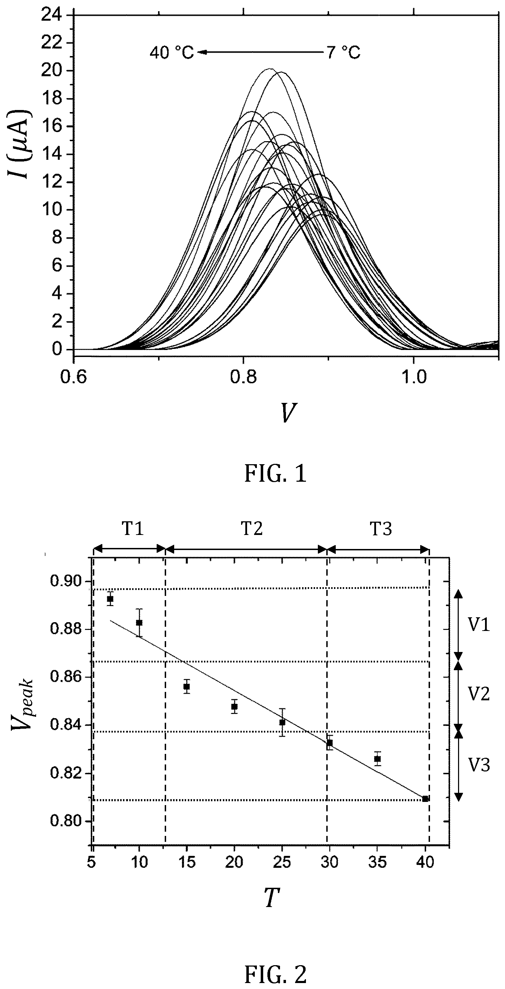

In this example, the temperature dependence of the voltammetric signal of cocaine is determined over a temperature range of from 7°C to 40°C. For this, a reference sample is prepared consisting of 0.5 mM cocaine in phosphate-buffered saline at a pH of 12. The reference voltammetric response was measured using square-wave voltammetric measurements and using a screen-printed carbon electrode as the active working electrode, a screen-printed carbon electrode as the counter electrode and a silver electrode as the reference electrode. The specific parameters of the square-wave voltametric measurements where as follow: step potential: 5 mV, frequency: 10 Hz, and amplitude: 25 mV. The temperature of the sample was controlled by performing the measurements in a temperature-controlled chamber. Measurements were performed at a temperature of the temperature-controlled chamber of 7 °C, 10 °C, 15 °C, 20 °C, 25 °C, 30 °C, 35 °C, and 40 °C. Before each measurement, the sample was stored in the temperature-controlled chamber to allow the sample temperature to equilibrate with the temperature of the temperature-controlled chamber. Therefore, it may be assumed that the sample temperature is equal to the temperature of the temperature-controlled chamber. At each temperature, the voltammetric response was measured three times, each time with different electrodes, although each time with the same type of electrodes. This was done to estimate an error in the results due to differences among different electrodes of a same type. Reference is made to FIG. 1, where the voltammetric signal, i.e., the current (I) in µA as dependent on voltage (V) in volt, of cocaine in the voltammetric response of the sample is shown for each of the three measurements performed at each temperature. On increasing the temperature, it is observed that the voltammetric signal of cocaine, and hence the peak voltage, shifts to lower voltages, as indicated by the arrow.

Reference is made to FIG. 2, where a voltage value (squares), corresponding to a peak voltage (in volt) of a measured voltammetric response, is plotted as dependent on sample temperature (in degrees Celsius). The error bar on each square is a standard error derived from the three measurements using different electrodes. A linear function is fitted to the voltage values (full line). Clearly, the line does not overlap with the voltage value as dependent on temperature within a standard error on the measurements. There is no linear relationship between the voltage value, i.e., the peak voltage, and the sample temperature.

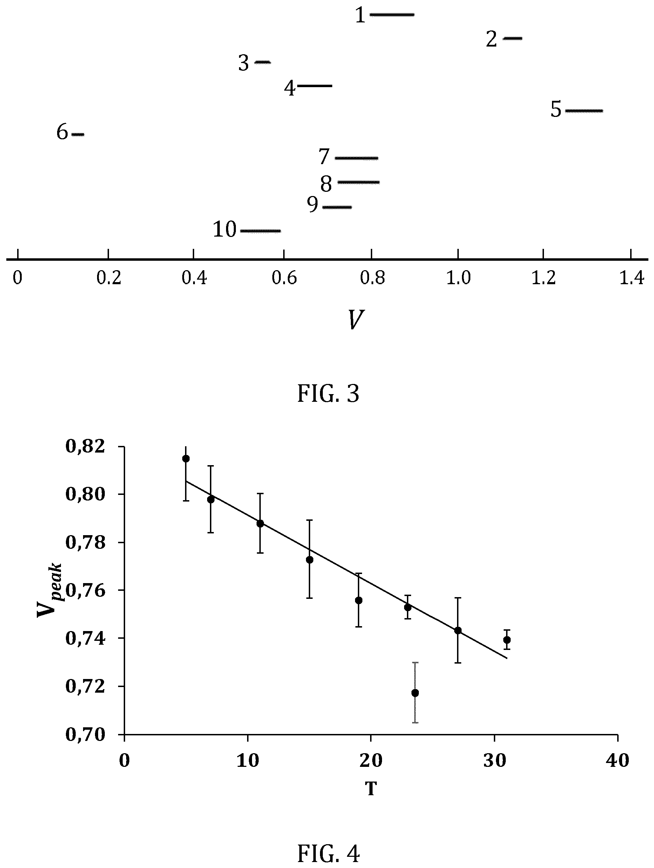

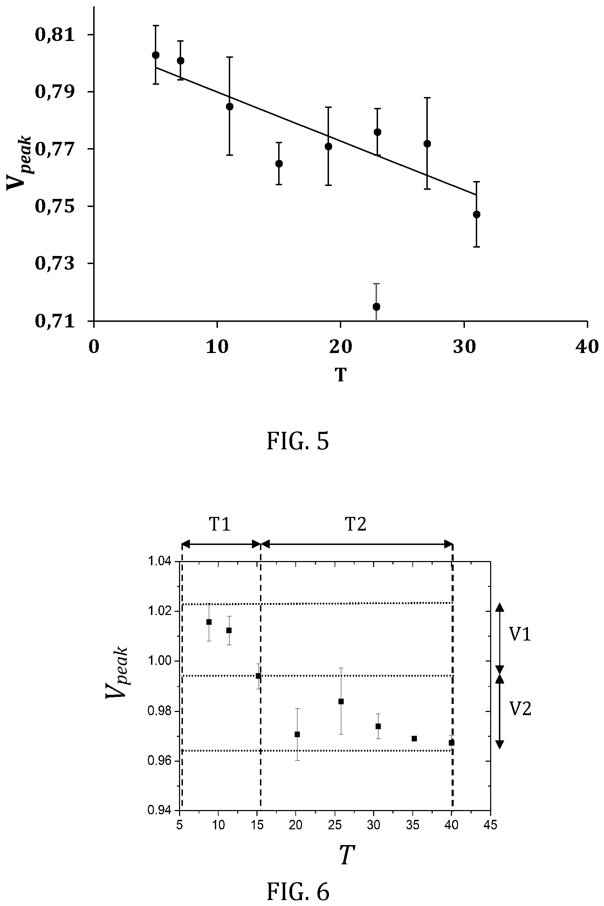

Reference is made to FIG. 3. From the voltammetric measurements on cocaine, a voltage range independent on temperature may be derived within which all voltage values i.e. peak values as dependent on temperature (i.e. of FIG. 2) fall. This voltage range has an upper limit of 0.887 and a lower limit of 0.808, and corresponds to the horizontal line 1. Each other horizontal line indicates such a voltage range for one of interferants 2 to 10, derived from measurements on the interferants similar to the measurements on cocaine described above. The peak voltage as dependent on temperature is in FIG. 4 shown for diltiazem and in FIG. 5 for hydroxyzine. The interferants are: 2: levamisole; 3: phenacetin; 4: lidocaine; 5: caffeine; 6: paracetamol; 7: diltiazem; 8: hydroxyzine; 9: procaine; and 10: benzocaine. As can be observed in FIG. 3, there is an overlap between the voltage range for cocaine 1 and the voltage ranges for diltiazem 7 and hydroxyzine 8. If the voltage range independent of temperature is used as a reference voltage range for determining the presence of an analyte in a sample, a false-positive determination may therefore result. That is, interferants diltiazem 7 or hydroxyzine 8 may be present in a sample and a voltammetric signal that is due to these interferants present in a voltammetric response of the sample may be wrongly attributed to cocaine. As may be verified by comparing FIG. 2 with FIG. 4 and FIG. 5, as the shift of the peak voltage with increasing temperature is, for each of cocaine and both interferants, towards lower voltages, there is no temperature at which the peak voltage of cocaine coincides with the peak voltage of one of the two interferants. In accordance with embodiments of the present invention, multiple (narrower) reference voltage ranges, each associated with a different temperature range may be set up, such that, for each of the reference voltage ranges, at a sample temperature in a temperature range associated with the reference voltage range, the peak voltage of one of the interferants does not fall in the reference voltage range.

In this example, a first reference voltage range associated with a first temperature range, a second reference voltage range associated with a second temperature range, and a third reference voltage range associated with a third temperature range were defined. As a rule, in this example, a different temperature range (or equivalently reference voltage range) is defined when the voltage values within the temperature range differ by more than 30 mV. Thereby, the voltage values within each temperature range are within 30 mV of each other. Expressed differently, associated with each temperature range, a core reference voltage range is defined with a width of 30mV within which the voltage values within the temperature range fall. For voltage values falling outside of the core reference voltage range, a different voltage value range, associated with a different temperature range and with a different reference voltage range, is defined. Within each temperature range, thereby, in this example, the shift of the voltammetric signal of the analyte as a result of sample temperature is limited to a maximum of 30 mV. The width of each reference voltage range may therefore be limited, as only a very small shift of the voltammetric signal due to the temperature has to be taken into account. The invention is however not limited to 30mV, and instead, narrower or wider core reference voltage ranges, or core reference voltage ranges each with a different width, may be defined.

In this example, it is chosen that all voltage values of the first temperature range fall in a first core reference voltage range with an upper limit of 0.898 V and a lower limit of 0.868 V (wherein there is the difference of 30 mV between upper and lower limit) indicated by dotted lines and double-sided arrow V1 in FIG. 3. In this example, the first temperature range T1 may for example have an upper limit of 12.5 °C and a lower limit of 5 °C (indicated by a dashed line). Thereby, each voltage value measured at a temperature falling within the first temperature range, falls within the first core reference voltage range. In this example, it is chosen that all voltage values of the second temperature range fall in a second core reference voltage range with an upper limit of 0.868 V (i.e. contiguous to the lower limit of the first temperature range) and a lower limit of 0.838 V, indicated by dotted lines and double-sided arrow V2 in FIG. 3. Thereby, in this example, as may be observed in FIG. 3, the second temperature range T2 may have a lower limit of 12.5 °C and an upper limit of 29.0 °C (indicated by a dashed line). In this example, it is chosen that all voltage values of the third temperature range fall in a third core reference voltage range with an upper limit of 0.838 V (i.e. contiguous to the lower limit of the second temperature range) and a lower limit of 0.808 V indicated by dotted lines and double-sided arrow V3 in FIG. 3. Thereby, in this example, as may be observed in FIG. 3, the third temperature range T3 may have a lower limit of 29.0 °C and an upper limit of 40 °C, indicated by the dashed line.

It has been observed by the inventors that the voltammetric signal may shift to higher voltages when, for example, an interferant (e.g. levamisole) is present in the sample. Therefore, in this example, the upper limit of each (e.g. first, second or third) reference voltage range is chosen to be 40 mV higher than the upper limit of the corresponding (e.g. first, second or third, respectively) core reference voltage range. It has been observed by the inventors that the voltammetric signal may shift to lower voltages when, for example, the concentration of cocaine is very low. Therefore, in this example, the lower limit of each reference voltage range is chosen to be 20 mV lower than the lower limit of the corresponding core reference voltage range. As a result, in this example, a first reference voltage range associated with the first temperature range has an upper limit of 0.938 V and a lower limit of 0.848 V. In this example, a second reference voltage range associated with the second temperature range has an upper limit of 0.908 V and a lower limit of 0.818 V. In this example, a third reference voltage range associated with the third temperature range has an upper limit of 0.878 V and a lower limit of 0.788 V. Thereby, in accordance with embodiments of the present invention, each reference voltage range comprises a plurality of different voltage values at which a voltammetric signal of the analyte can be detected at a temperature falling in the temperature range associated with the reference voltage range. The reference voltage ranges and the associated temperature ranges for cocaine are, however, only examples, and the invention is not limited thereto. For example, as is clear from FIG. 3, alternative ranges may be chosen based on the measured data. Furthermore, instead of voltammetric responses of pure samples of cocaine, interferants may be added to the sample, measurements may be performed at a different pH, or the concentration of cocaine in the sample may be different. This may influence the voltage at which the peak voltage at each temperature occurs, which in turn may influence the reference voltage ranges and associated temperature ranges that may be chosen based on the measured data.

Reference is made to FIG. 6, which is a plot of peak voltages as dependent on temperature for ketamine in a sample at a pH of 12, determined using similar measurements as above for cocaine. Also, for ketamine, a nonlinear dependence of the peak voltage as dependent on temperature may be observed. In this example, using these data, a first reference voltage range associated with a first temperature range, and a second reference voltage range associated with a second temperature range is determined. Similarly, as for cocaine above, a different temperature range (or equivalently reference voltage range) is defined when the voltage values within the temperature range differ by more than 30 mV, although, instead, another difference could be chosen, e.g., a difference situated in the range from 10 mV to 50 mV, such as from 20 to 40 mV. In this example, it is determined that all voltage values of the first temperature range fall in a first core reference voltage range V1 with an upper limit of 1.024 V and a lower limit of 0.994 V. In this example, the first temperature range T1 may, for example, have an upper limit of from 14 to 18 °C, e.g., 16 °C. In this example, it is chosen that all voltage values of the second temperature range fall in a second core reference voltage range V2 with an upper limit of 0.994 V (i.e. contiguous to the lower limit of the first temperature range) and a lower limit of 0.964 V. Thereby, in this example, the second temperature range T2 may have a lower limit of from 14 to 18 °C, e.g., 16 °C, preferably contiguous to the upper limit of the first temperature range, and an upper limit of from 27 to 33 °C, e.g., 29.0 °C. The chosen core reference voltage ranges and temperature ranges are indicated in FIG. 6. These values are only an example, and also based on the presented data different core reference voltage ranges and temperature ranges may be chosen.

In this example, a first reference voltage range associated with the first temperature range has an upper limit of from 1.024V to 1.084V, e.g., 1.064 V and a lower limit of from 0.954 V to 0.994 V, e.g., 0.974 V. In this example, a second reference voltage range associated with the second temperature range has an upper limit of from 1.014 V to 1.054 V, e.g., 1.034 V and a lower limit of from 0.924 V to 0.964 V, e.g., 0.944 V. These values are only an example, and also based on the presented data different reference voltage ranges may be chosen.

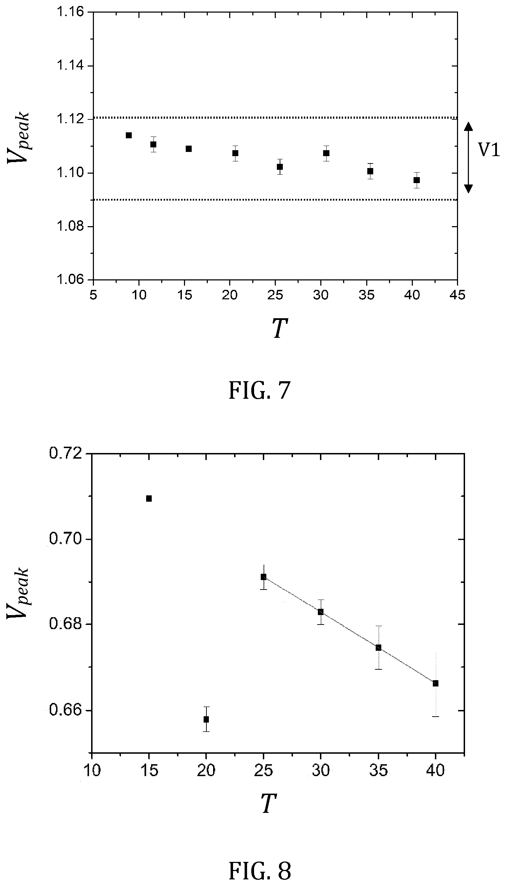

Reference is made to FIG. 7, which is a plot of peak voltages as dependent on temperature for 3,4-methylenedioxymethamphetamine, determined using similar measurements as above for cocaine, but at a pH of 5. Clearly, a nonlinear dependence of the peak voltage as dependent on temperature may be observed. The shift is, however, relatively small. Therefore, when, as above, a different temperature range (or equivalently reference voltage range) is defined when the voltage values within the temperature range differ by more than 30 mV (i.e., V1 in FIG. 7), only a single reference voltage range may be defined. If, instead, a different temperature range (or equivalently reference voltage range) is defined when the voltage values within the temperature range differ by more than, e.g., from 10 to 15 mV, at least two reference voltage ranges may be defined, in accordance with embodiments of the present invention.

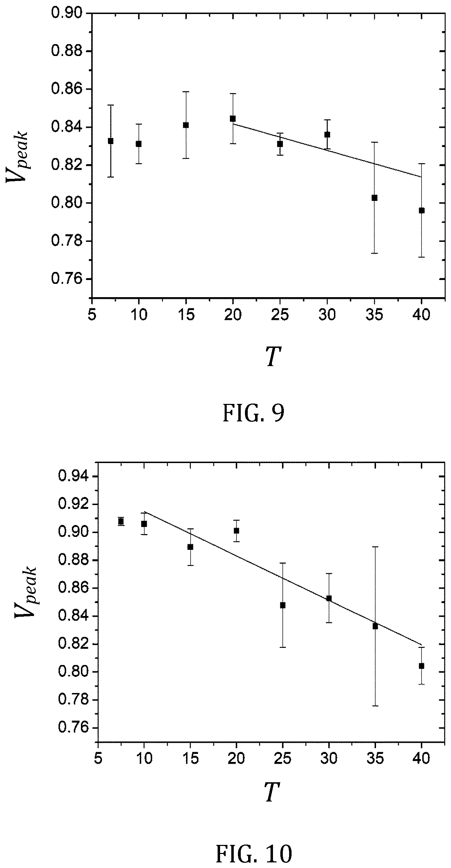

Reference is made to FIG. 8, which is a plot of peak voltages as dependent on temperature for amphetamine determined using similar measurements as above for cocaine. Reference is made to FIG. 9, which is a plot of peak voltages as dependent on temperature for heroin determined using similar measurements as above for cocaine. Reference is made to FIG. 10, which is a plot of peak voltages as dependent on temperature for methamphetamine determined using similar measurements as above for cocaine. For each of the narcotics, there is clearly a nonlinear relationship between the peak voltage and the sample temperature.

### Example 2: determining the presence of an analyte in a sample at a sample temperature

In an example in accordance with embodiments of the present invention, 173 unknown powder samples are analyzed to determine the presence of an analyte, in this example cocaine, in the powder. The 173 powders have different contents: each of the samples comprises at least cocaine, and possibly, in addition, interferants. In this example, for each powder, a sample comprising a buffered aqueous solution at pH 12 comprising the unknown powder is prepared. The samples have different temperatures. A sample temperature is determined. Subsequently, a reference voltage range, associated with a temperature range in which the sample temperature falls, is selected. In this example, the reference voltage range is selected from the first, second, and third reference voltage range, associated with, respectively, the first, second, and third temperature range, as determined above in Example 1 for cocaine, except that the lower limit of the second reference voltage range in this example is 0.808 V (instead of 0.818 V). For each of the 173 samples, a voltammetric response is measured at least over the reference voltage range selected for the sample. For each sample, the voltammetric response is measured with screen-printed carbon electrodes and a silver reference electrode.

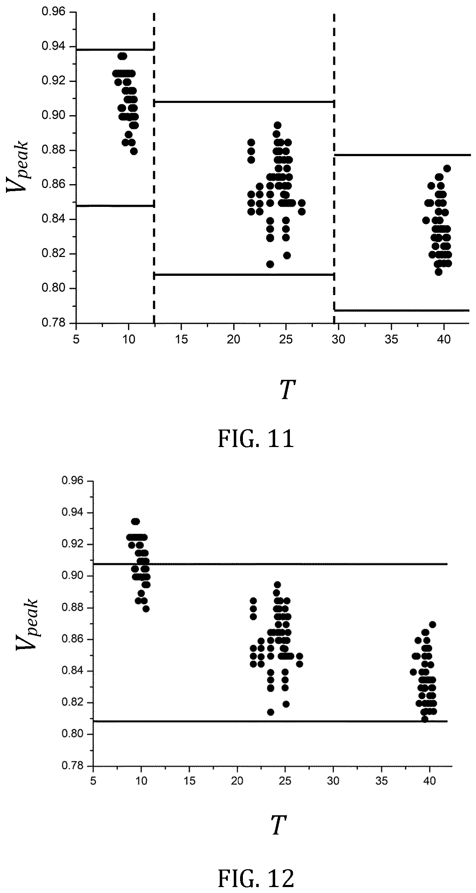

Reference is made to FIG. 11. In the voltammetric response of each sample, at least a peak voltage falling in the reference voltage range is observed. The peak voltage of each sample is plotted in FIG. 11 at the sample temperature. The first, second, and third reference voltage ranges (between full lines, from left to right) associated with, respectively, the first, second, and third temperature ranges (separated by dashed lines, from left to right) are also indicated in FIG. 11. In this example, for each of the 173 samples, the presence of cocaine is correctly indicated. Thereby, this example, which is in accordance with embodiments of the present invention, enables a >99% accuracy, by determining that cocaine is present in each of the 173 samples. At the same time, no false-positives are determined e.g. due to the presence of a voltage peak of an interferant falling in the reference voltage range.

Reference is made to FIG. 12. As a comparison, instead of the three reference voltage ranges used above, each associated with a different temperature range, which is in accordance with embodiments of the present invention, a single voltage range is used, which is not in accordance with embodiments of the present invention. In FIG. 12, the single voltage range corresponds to the second reference voltage range from FIG. 11. As can be observed in FIG. 12, a large fraction of the peak voltages at a sample temperature of approximately 10 °C does not fall in the single reference voltage range. Thereby, the accuracy of the determination of the presence of cocaine in the sample, in this case, is only 84%, which is much lower than the >99% that was reached with an example in accordance with embodiments of the present invention above. Although a single reference voltage range could be used that is wider, so that 100% of the peak voltages falls with the single reference voltage range, this is at the risk of an increased probability of a false-positive determination of cocaine in a sample.

A further method in accordance with embodiments of the present invention has been performed for ketamine. For ketamine, also a >99% accuracy is reached when a method in accordance with embodiments of the present invention was used, whereas accuracy of only 75% is reached when, instead, a single reference voltage range was used.

It is to be understood that although preferred embodiments, specific constructions, and configurations, as well as materials, have been discussed herein for devices according to the present invention, various changes or modifications in form and detail may be made without departing from the scope of the invention as claimed.

## Claims

1. A method for determining the presence of an analyte (1) in a sample, distinguished from the possible presence of an interferant (2-10) in the sample, at a sample temperature, the method comprising:
- selecting a reference voltage range (V1, V2, V3) amongst at least a first reference voltage range (V1) and a second reference voltage range (V2), wherein the first reference voltage range (V1) is associated with a first temperature range (T1) and the second reference voltage range (V2) is associated with a second temperature range (T2), wherein the reference voltage range (V1, V2, V3) which is selected is associated with the temperature range (T1, T2, T3) in which the sample temperature falls, each reference voltage range (V1, V2, V3) comprising a plurality of different peak voltage values of a voltammetric signal of the analyte (1) at a temperature falling in the temperature range (T1, T2, T3) associated with the reference voltage range (V1, V2, V3), wherein each reference voltage range (V1, V2, V3) is adapted so that a peak voltage value of a voltammetric signal of the interferant (2-10) at a temperature falling in the temperature range (T1, T2, T3) associated with the reference voltage range (V1, V2, V3) does not fall within the reference voltage range (V1, V2, V3),
- measuring a voltammetric response of the sample (1) at least across the selected reference voltage range (V1, V2, V3), and
- determining whether the analyte (1) is present in the sample by determining whether a peak voltage of the measured voltammetric response falls within the selected reference voltage range (V1, V2, V3).

2. The method according to claim 1, wherein a difference between an upper limit and a lower limit of each of the reference voltage ranges (V1, V2, V3) is of from 30 mV to 200 mV, preferably of from 60 mV to 120 mV.

3. The method according to any of the previous claims, wherein the reference voltage ranges (V1, V2, V3) cover a temperature range (T1, T2, T3) of from 10 °C to 30 °C, preferably of from 1 °C to 40.

4. The method according to any of the previous claims, wherein the analyte (1) is a narcotic.

5. The method according to claim 4, wherein the narcotic is cocaine, amphetamine, or ketamine, and wherein the pH of the sample is from 10 to 13.

6. The method according to claim 4 or 5, wherein the narcotic is cocaine, wherein selecting a reference voltage range (V1, V2, V3) is performed amongst a first (V1), a second (V2), and a third reference voltage range (V3), wherein the first reference voltage range (V1) is associated with a first temperature range (T1) having an upper limit of from 11.5 °C to 13.5 °C, wherein the second reference voltage range (V2) is associated with a second temperature range (T2) having a lower limit higher but contiguous to the upper limit of the first temperature range (T1) and an upper limit of from 28.0 °C to 30.0 °C, and wherein the third reference voltage range (V3) is associated with a third temperature range (T3) having a lower limit higher but contiguous to the upper limit of the second temperature range (T2).

7. The method according to claim 6, wherein the first reference voltage range (V1) has a lower limit of from 0.834 to 0.862 V and an upper limit of from 0.924 to 0.952 V, wherein the second reference voltage range (V2) has a lower limit of from 0.794 V to 0.832 V and an upper limit of from 0.894 V to 0.922 V, and wherein the third reference voltage range (V3) has a lower limit of from 0.774 V to 0.802 V and an upper limit of from 0.864 V to 0.892 V.

8. The method according to any of the previous claims, wherein the voltammetric response is measured using a screen-printed carbon working electrode.

9. The method according to any of the previous claims, wherein the voltammetric response is measured using square wave voltammetry.

10. The method according to any of the previous claims, wherein each reference voltage range (V1, V2, V3) is an oxidation voltage range.

11. A data processing apparatus adapted for carrying out the steps of the method of any of the previous claims, comprising:
an inlet for a sample,
a unit for acquiring the temperature of the sample,
a memory for storing at least a first reference voltage range (V1), a second reference voltage range (V2), and temperature ranges (T1, T2) associated therewith,
a selection unit for selecting a reference voltage range (V1, V2, V3), amongst those stored in the memory, associated with a temperature range (T1, T2, T3) in which the sample temperature falls,
a voltammetric unit for measuring a voltammetric response of the sample, and
an analyzing unit for determining whether the analyte (1) is present in the sample by analyzing whether the voltammetric signal of the analyte (1) can be detected in the measured voltammetric response.

## Patentansprüche

1. Ein Verfahren zum Bestimmen des Vorhandenseins eines Analyten (1) in einer Probe, das sich vom möglichen Vorhandensein einer Störsubstanz (2-10) in der Probe bei einer Probentemperatur unterscheidet, wobei das Verfahren umfasst:
- Auswählen eines Referenzspannungsbereichs (V1, V2, V3) aus mindestens einem ersten Referenzspannungsbereich (V1) und einem zweiten Referenzspannungsbereich (V2), wobei der erste Referenzspannungsbereich (V1) einem ersten Temperaturbereich (T1) zugeordnet ist, und der zweite Referenzspannungsbereich (V2) einem zweiten Temperaturbereich (T2) zugeordnet ist, wobei der Referenzspannungsbereich (V1, V2, V3), der ausgewählt wird, dem Temperaturbereich (T1, T2, T3) zugeordnet ist, in dem die Probentemperatur abfällt, wobei jeder Referenzspannungsbereich (V1, V2, V3) eine Vielzahl von verschiedenen Spitzenspannungswerten eines voltammetrischen Signals des Analyten (1) bei einer abfallenden Temperatur in dem Temperaturbereich (T1, T2, T3), der dem Referenzspannungsbereich (V1, V2, V3) zugeordnet ist, umfasst, wobei jeder Referenzspannungsbereich (V1, V2, V3) angepasst ist, sodass ein Spitzenspannungswert eines voltammetrischen Signals der Störsubstanz (2-10) bei einer abfallenden Temperatur im Temperaturbereich (T1, T2, T3), die dem Referenzspannungsbereich (V1, V2, V3) zugeordnet ist, nicht innerhalb des Referenzspannungsbereichs (V1, V2, V3) fällt,
- Messen einer voltammetrischen Reaktion der Probe (1) mindestens über den ausgewählten Referenzspannungsbereich (V1, V2, V3), und
- Bestimmen, ob der Analyt (1) in der Probe vorhanden ist, durch Bestimmen, ob eine Spitzenspannung der gemessenen voltammetrischen Reaktion innerhalb des ausgewählten Referenzspannungsbereichs (V1, V2, V3) fällt.

2. Das Verfahren nach Anspruch 1, wobei eine Differenz zwischen einer Obergrenze und einer Untergrenze jedes der Referenzspannungsbereiche (V1, V2, V3) 30 mV bis 200 mV, vorzugsweise 60 mV bis 120 mV beträgt.

3. Das Verfahren nach einem der vorstehenden Ansprüche, wobei die Referenzspannungsbereiche (V1, V2, V3) einen Temperaturbereich (T1, T2, T3) von 10 °C bis 30 °C, vorzugsweise von 1 °C bis 40 abdecken.

4. Das Verfahren nach einem der vorstehenden Ansprüche, wobei der Analyt (1) ein Narkotikum ist

5. Das Verfahren nach Anspruch 4, wobei das Narkotikum Kokain, Amphetamin, oder Ketamin ist, und wobei der pH-Wert der Probe von 10 bis 13 reicht.

6. Das Verfahren nach Anspruch 4 oder 5, wobei das Narkotikum Kokain ist, wobei Auswählen eines Referenzspannungsbereichs (V1, V2, V3) aus einem ersten (V1), einem zweiten (V2), und einem dritten Referenzspannungsbereich (V3) durchgeführt wird, wobei der erste Referenzspannungsbereich (V1) einem ersten Temperaturbereich (T1) zugeordnet ist, der eine Obergrenze von 11,5 °C bis 13,5 °C aufweist, wobei der zweite Referenzspannungsbereich (V2) einem zweiten Temperaturbereich (T2) zugeordnet ist, der eine Untergrenze aufweist, die höher, aber angrenzend an die Obergrenze des ersten Temperaturbereichs (T1) ist und eine Obergrenze von 28,0 °C bis 30,0 °C aufweist, und wobei der dritte Referenzspannungsbereich (V3) einem dritten Temperaturbereich (T3) zugeordnet ist, der eine Untergrenze höher, aber angrenzend an die Obergrenze des zweiten Temperaturbereichs (T2) aufweist.

7. Das Verfahren nach Anspruch 6, wobei der erste Referenzspannungsbereich (V1) eine Untergrenze von 0,834 bis 0,862 V und eine Obergrenze von 0,924 bis 0,952 V aufweist, wobei der zweite Referenzspannungsbereich (V2) eine Untergrenze von 0,794 V bis 0,832 V und eine Obergrenze von 0,894 V bis 0,922 V aufweist, und wobei der dritte Referenzspannungsbereich (V3) eine Untergrenze von 0,774 V bis 0,802 V und eine Obergrenze von 0,864 V bis 0,892 V aufweist.

8. Das Verfahren nach einem der vorstehenden Ansprüche, wobei die voltammetrische Reaktion unter Verwendung einer Siebdruck-Kohlenstoffarbeitselektrode gemessen wird.

9. Das Verfahren nach einem der vorstehenden Ansprüche, wobei die voltammetrische Reaktion unter Verwendung einer Rechteckwellenvoltammetrie gemessen wird.

10. Das Verfahren nach einem der vorstehenden Ansprüche, wobei jeder Referenzspannungsbereich (V1, V2, V3) ein Oxydationsspannungsbereich ist.

11. Eine Datenverarbeitungseinrichtung, die zum Ausführen der Schritte des Verfahrens nach einem der vorstehenden Ansprüche angepasst ist, umfassend:
einen Einlass für eine Probe,
eine Einheit zum Erfassen der Temperatur der Probe,
einen Speicher zum Speichern mindestens eines ersten Referenzspannungsbereichs (V1), eines zweiten Referenzspannungsbereichs (V2), und von diesen zugeordneten Temperaturbereichen (T1, T2),
eine Auswahleinheit zum Auswählen eines Referenzspannungsbereichs (V1, V2, V3) aus jenen, die im Speicher gespeichert sind, die einem Temperaturbereich (T1, T2, T3) zugeordnet sind, in den die Probentemperatur fällt,
eine voltammetrische Einheit zum Messen einer voltammetrischen Reaktion der Probe, und
eine Analyseeinheit zum Bestimmen, ob der Analyt (1) in der Probe vorhanden ist, durch Analysieren, ob das voltammetrische Signal des Analyten (1) in der gemessenen voltammetrischen Reaktion nachgewiesen werden kann.

## Revendications

1. Une méthode pour déterminer la présence d'un analyte (1) dans un échantillon, distinguée de la présence possible d'un interférant (2-10) dans l'échantillon, à une température d'échantillon, la méthode comprenant :
• la sélection d'une plage de tension de référence (V1, V2, V3) parmi au moins une première plage de tension de référence (V1) et une seconde plage de tension de référence (V2), dans laquelle la première plage de tension de référence (V1) est associée à une première plage de température (T1) et la seconde plage de tension de référence (V2) est associée à une seconde plage de température (T2), dans laquelle la plage de tension de référence (V1, V2, V3) qui est sélectionnée est associée à la plage de température (T1, T2, T3) dans laquelle tombe la température de l'échantillon, chaque plage de tension de référence (V1, V2, V3) comprenant une pluralité de valeurs de tension de pic différentes d'un signal voltammétrique du dit analyte (1) à une température tombant dans la plage de température (T1, T2, T3) associée à la dite plage de tension de référence (V1, V2, V3), dans laquelle chaque plage de tension de référence (V1, V2, V3) est adaptée de sorte qu'une valeur de tension de pic d'un signal voltammétrique du dit interférant (2-10) à une température tombant dans la plage de température (T1, T2, T3) associée à la dite plage de tension de référence (V1, V2, V3) ne tombe pas dans la dite plage de tension de référence (V1, V2, V3),
• la mesure d'une réponse voltammétrique du dit échantillon (1) au moins à travers la dite plage de tension de référence sélectionnée (V1, V2, V3), et
• la détermination de la présence du dit analyte (1) dans l'échantillon en déterminant si une tension de pic de la dite réponse voltammétrique mesurée tombe dans la dite plage de tension de référence sélectionnée (V1, V2, V3).

2. La méthode selon la revendication 1, dans laquelle une différence entre une limite supérieure et une limite inférieure de chacune des dites plages de tension de référence (V1, V2, V3) est de 30 mV à 200 mV, de préférence de 60 mV à 120 mV.

3. La méthode selon l'une quelconque des revendications précédentes, dans laquelle les dites plages de tension de référence (V1, V2, V3) couvrent une plage de température (T1, T2, T3) de 10 °C à 30 °C, de préférence de 1 °C à 40 °C.

4. La méthode selon l'une quelconque des revendications précédentes, dans laquelle le dit analyte (1) est un narcotique.

5. La méthode selon la revendication 4, dans laquelle le dit narcotique est la cocaïne, l'amphétamine, ou la kétamine, et dans laquelle le pH de l'échantillon est de 10 à 13.

6. La méthode selon la revendication 4 ou 5, dans laquelle le dit narcotique est la cocaïne, dans laquelle la sélection d'une plage de tension de référence (V1, V2, V3) est effectuée parmi une première (V1), une seconde (V2), et une troisième plage de tension de référence (V3), dans laquelle la première plage de tension de référence (V1) est associée à une première plage de température (T1) ayant une limite supérieure de 11,5 °C à 13,5 °C, dans laquelle la seconde plage de tension de référence (V2) est associée à une seconde plage de température (T2) ayant une limite inférieure plus élevée mais contiguë à la limite supérieure de la première plage de température (T1) et une limite supérieure de 28,0 °C à 30,0 °C, et dans laquelle la troisième plage de tension de référence (V3) est associée à une troisième plage de température (T3) ayant une limite inférieure plus élevée mais contiguë à la limite supérieure de la seconde plage de température (T2).

7. La méthode selon la revendication 6, dans laquelle la première plage de tension de référence (V1) a une limite inférieure de 0,834 à 0,862 V et une limite supérieure de 0,924 à 0,952 V, dans laquelle la seconde plage de tension de référence (V2) a une limite inférieure de 0,794 V à 0,832 V et une limite supérieure de 0,894 V à 0,922 V, et dans laquelle la troisième plage de tension de référence (V3) a une limite inférieure de 0,774 V à 0,802 V et une limite supérieure de 0,864 V à 0,892 V.

8. La méthode selon l'une quelconque des revendications précédentes, dans laquelle la dite réponse voltammétrique est mesurée en utilisant une électrode de travail en carbone sérigraphiée.

9. La méthode selon l'une quelconque des revendications précédentes, dans laquelle la dite réponse voltammétrique est mesurée en utilisant la voltammétrie à onde carrée.

10. La méthode selon l'une quelconque des revendications précédentes, dans laquelle chaque plage de tension de référence (V1, V2, V3) est une plage de tension d'oxydation.

11. Un appareil de traitement de données adapté pour effectuer les étapes de la méthode de l'une quelconque des revendications précédentes, comprenant :
une entrée pour un échantillon,
une unité pour acquérir la température de l'échantillon,
une mémoire pour stocker au moins une première plage de tension de référence (V1), une seconde plage de tension de référence (V2), et les plages de température (T1, T2) associées à celles-ci,
une unité de sélection pour sélectionner une plage de tension de référence (V1, V2, V3), parmi celles stockées dans la mémoire, associée à une plage de température (T1, T2, T3) dans laquelle tombe la température de l'échantillon,
une unité voltammétrique pour mesurer une réponse voltammétrique de l'échantillon, et
une unité d'analyse pour déterminer si le dit analyte (1) est présent dans l'échantillon en analysant si le signal voltammétrique du dit analyte (1) peut être détecté dans la dite réponse voltammétrique mesurée.
